# EUROPEAN PATENT APPLICATION

(11) **EP 1 552 782 A1**
(43) Date of publication of application: **13.07.2005**
(21) Application number: 05000135.3
(22) Date of filing: 05.01.2005
(51) Int. Cl.: A61B 5/00, A61N 1/30

(54) **Analyte extracting device, kit and method, and blood sugar measuring device and method**

(30) Priority: 09.01.2004 JP 2004003826
(71) Applicant: Sysmex Corporation, Kobe-shi, Hyogo 651-0073 (JP)
(72) Inventor: Okada, Seiki, Kobe-shi Hyogo 651-0079 (JP); Maekawa, Yasunori, Kobe-651-2277 (JP); Sawa, Kenichi, Amagasaki-shi Hyogo 661-0024 (JP); Sato, Toshiyuki, Nishinomiya-shi Hyogo 662-0024 (JP); Nagaoka, Kanako, Kobe 654-0031 (JP); Hagino, Kei, Kobe 651-2273 (JP)
(74) Representative: HOFFMANN - EITLE

(57) **Abstract**

An extracting kits for extracting analyte through the skin of a living body are disclosed that comprises an inserting device comprising needles for forming a plurality of extraction holes in the skin through the stratum corneum but not reaching the subcutaneous tissue and an extracting device (36) for extracting the analyte into a liquid supplied into the extraction holes by supplying the liquid into the plurality of extraction holes. An extracting device, blood sugar measuring device (31), extracting method, and blood sugar measuring method are also described.

## Description

### FIELD OF THE INVENTION

The present invention relates to an extracting kit for extracting analyte through the skin of a living body, extracting device, blood sugar measuring device, extracting method, and blood sugar measuring method.

### BACKGROUND

Devices for extracting glucose through the skin using reverse iontophoresis (for example, US Patent Publication No. 6,233,471) and devices for extracting glucose using a hollow needle (for example WIPO Patent Pamphlet No. WO95/010223) have been disclosed as percutaneous analyte extraction assemblies for extracting glucose contained in the tissue fluids of living bodies through the skin.

Although the reverse iontophoresis described in US Patent Publication No. 6,233,471 allows extraction of glucose through intact skin without invasion to the skin, it provides painful discomfort to the subject, because it is necessary to apply a higher voltage (greater current) for extraction of glucose sufficient for analysis in a short period of time. For example, the density of electric current flowing through the skin may be in the range of approximately 0.01 - 0.5 mA/cm² according to US Patent Publication No. 6,233,471, but it is necessary to apply a significantly higher voltage for making such an amount of current flow through the intact skin, which is higher in electric resistance.

US Patent Publication No. 6,233,471 also proposes a method of extracting biological samples by combined use of the reverse iontophoresis and a microneedle for puncture. However, the method causes both the pains of electric flow and microneedle puncture in the subject.

Alternatively, WIPO Patent Pamphlet No. WO95/010223 discloses a device for inserting a hollow needle to an extent that it does not penetrate through the corium and extracting fluids under a negative pressure or by capillary phenomenon. However, the device, which extracts glucose through a hole of needle under a negative pressure or by capillary phenomenon, carried the problems in stability and extraction speed.

### SUMMARY

The scope of the present invention is defined sole by the appended claims, and is not affected to any degree by the statements within this summary.

The present invention provides a new extracting method that overcomes the problems above.

A first aspect of the present invention relates to an extracting kit for extracting analyte through the skin of a living body, the extracting kit including an inserting device comprising needles for forming a plurality of extraction holes in the skin through the stratum corneum but not reaching the subcutaneous tissue and an extracting device for extracting the analyte into a liquid supplied into the extraction holes by supplying the liquid into the plurality of extraction holes.

A second aspect of the present invention relates to an extracting device for extracting analyte through the skin of a living body, the extracting device including a power source for applying a voltage for transferring analyte extracted into extraction holes formed on the skin out of the extraction holes and a checking means for checking the state of extraction hole formation.

A third aspect of the present invention relates to a blood sugar measuring device for extracting glucose through the skin of a living body and analyzing the glucose, the measuring device including an input unit for inputting an initial blood sugar value; a power source for transferring glucose extracted into extraction holes formed on the skin out of the extraction holes; a detecting unit for detecting signals based on the extracted glucose; and a control unit for calculating a blood sugar value from the signal detected by the detecting unit; wherein the control unit calculates glucose concentration from the signal detected by the detecting unit, and calculates an assay time blood sugar value based on the glucose concentration and the initial blood sugar value input by the input unit.

A fourth aspect of the present invention relates to an extracting method for extracting analyte through the skin of a living body, the method including an extraction hole formation step of forming a plurality of extraction holes in the skin through the stratum corneum but not reaching the subcutaneous tissue; and an extraction step of extracting the analyte into a liquid supplied to the extraction holes by supplying the liquid into the plurality of extraction holes.

A fifth aspect of the present invention relates to an extracting method for extracting analyte through the skin of a living body, the method including an extraction hole formation step for forming a plurality of extraction holes in the skin for the extraction of analyte a checking step for checking the state of extraction hole formation; and a transferring step of transferring the analyte extracted into the extraction holes out of the extraction holes if the extraction holes are judged to be formed sufficiently in the checking step.

A sixth aspect of the present invention relates to a blood sugar measuring method for extracting glucose through the skin of a living body and analyzing the glucose, the method including an extraction hole formation step for forming an extraction hole in the skin for extracting analyte an initial blood sugar value input step for inputting an initial blood sugar value an electric field application step of applying a voltage for migrating glucose extracted into the extraction hole out of the extraction hole a detection step for detecting a signal based on the glucose extracted in the extracting device; and a calculation step for calculating a blood sugar value from a signal detected in the detection step; wherein, in the calculation step, a glucose concentration is calculated from the signal detected in the detection step, and an assay time blood sugar value is calculated based on the glucose concentration and an initial blood sugar value input in the initial blood sugar value input step.

A seventh aspect of the present invention relates to an extracting kit for extracting analyte through the skin of a living body, the extracting kit including an inserting device comprising a needle for forming an extraction hole in the skin through the stratum corneum but not reaching the subcutaneous tissue and an extracting device for extracting analyte outside of the extraction hole and placed on the skin where the extraction hole is formed.

A eighth aspect of the present invention relates to an extracting method for extracting analyte through the skin of a living body, the method including an extraction hole formation step of forming an extraction hole in the skin through the stratum corneum but not reaching the subcutaneous tissue and an extraction step of extracting analyte outside of the extraction hole.

### BRIEF DESCRIPTION OF THE DRAWINGS

Figs. 1(a) through 1(c) illustrate the method of using the extracting kit and blood sugar measuring device of an embodiment of the present invention;
Fig. 2(a) and 2(b) illustrate the inserting device of the extracting kit of the embodiment;
Fig. 3 is a block diagram briefly showing the structure of the blood sugar measuring device of an embodiment of the present invention;
Fig. 4 is a perspective view of another embodiment of the inserting device;
Fig. 5 is a schematic view showing a cross section of the skin of a person;
Fig. 6 is a flow chart showing the operating sequence performed each day by a subject using the extracting kit and blood sugar measuring device of the embodiment of the present invention;
Fig. 7 is a flow chart showing details of step S2 for checking the state of the extraction hole in the flow chart of Fig. 6;
Fig. 8 is a flow chart showing details of step S5 for extracting glucose, and calculating the initial glucose concentration, average current value, and glucose concentration per unit of initial current; and
Fig. 9 is a flow chart showing details of step S8 for measuring a blood sugar value using the blood sugar measuring device of an embodiment of the present invention.
Fig. 10 is a schematic view illustrating the cross section of the skin in the extraction region where extraction holes are formed.
Fig. 11 is a schematic view illustrating the cross section of the skin in the extraction region when physiological saline is fed to the extraction holes.
Fig. 12 is a schematic view illustrating the cross section of the skin in the extraction region when the body fluid extracted into the extraction holes from the corium is diffusing in physiological saline.
Fig. 13 is a schematic view illustrating the cross section of the skin in the extraction region when the body fluid extracted into the physiological saline in extraction holes is traveling toward an extraction unit by application of an electric field.

### DETAILED DESCRIPTION OF THE PREFERRED EMBODIEMNTS

The preferred embodiments of the present invention are described hereinafter with reference to the drawings.

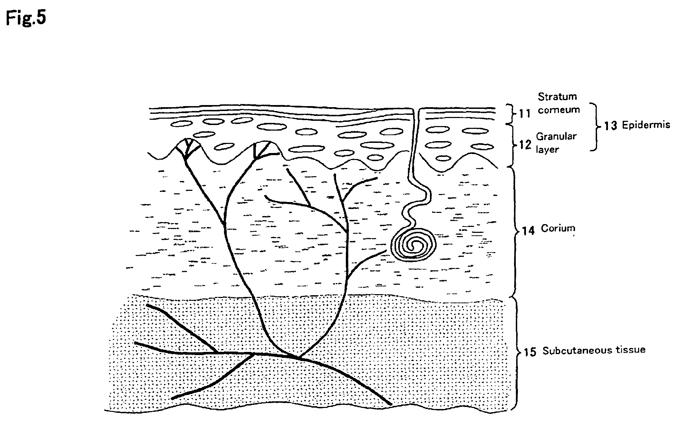

Fig. 5 schematically shows a cross section of the skin of a person. As shown in the drawing, the epidermis 13 is in direct contact with the atmosphere on the outermost surface of the skin of the person. The epidermis 13 includes a stratum corneum 11 which has a high electrical resistance value, and a granular layer 12 positioned on the underside of the stratum corneum 11 and the like. The corium 14 is below the epidermis 13, and subcutaneous tissue 15 is beneath the corium 14.

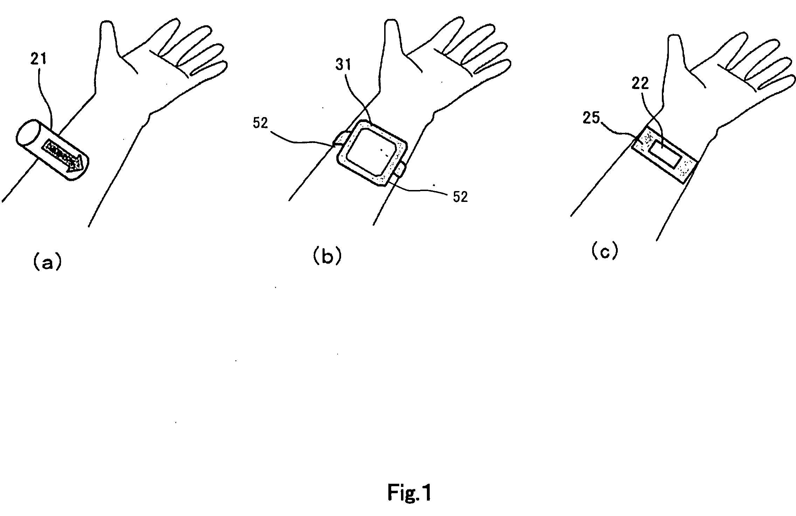

Figs. 1(a) through 1(c) illustrate the method of using the extracting kit (including the inserting device 21 and blood sugar measuring device (extracting device)) 31 of an embodiment of the present invention. The inserting device 21 forms, in the extraction region, an extraction hole at least passing through the stratum corneum and reaching to the middle or lower part (subcutaneous tissue side) of the conium 14 of the skin of a subject but not penetrating to the subcutaneous tissue 15. The blood sugar measuring device 31 has the external appearance of a wristwatch and is worn around the forearm of the subject, and is fastened in place by means of a band 52. A marker 25 is a sheet adhered to the skin which functions as a mark for specifying the region 22 of the skin in which the extraction hole is formed by the inserting device 21.

From the perspective of alleviating the painful discomfort experienced by the subject, the extraction hole formed by the inserting device 21 may be a hole passing through the stratum corneum 11 and penetrating to the upper part (skin surface side) of the corium 14 but not reaching the middle of the corium 14, or may be a hole passing through the stratum corneum 11 and reaching the granular layer 12, but not penetrating to the corium 14.

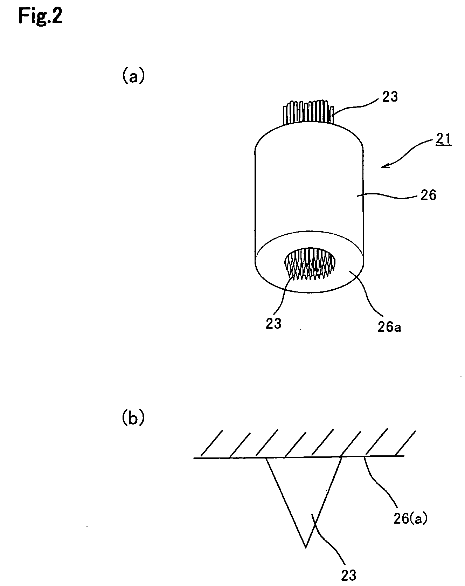

Fig. 2 (a) is a perspective view of the inserting device 21. The inserting device 21 is structured as a plurality of needles 23 bundled together by a holder 26; the holder 26 has an external diameter of 10 mm, internal diameter of 3 mm, and holds 30 needles 23; the length from the edge 26a of the holder 26 to the needles 23 is 300 µm. Fig. 2 (b) is a view illustrating a needle 23 sticking out of the edge 26a. The portion of the needle 23 sticking out of the edge 26a is conical in shape, and the diameter at the area closest to the edge 26, i.e., the base of the cone, is 160 µm.
The size of the extraction region of the skin in which the extraction hole is formed is determined in accordance with the size of the extracting device; when the extracting region is large, and inserting device 21 having a bundle of many needles may be used, or the inserting device 21 may be moved to a plurality of positions to apply pressure on the skin. The length of the needle 23 part of the inserting device 21, which extends from the edge 26a of the holder 26, is desirably greater than approximately 20 µm and less than approximately 1,000 µm so as to form an extraction hole that passes through the stratum corneum 11 but does not penetrate to the subcutaneous tissue 15.

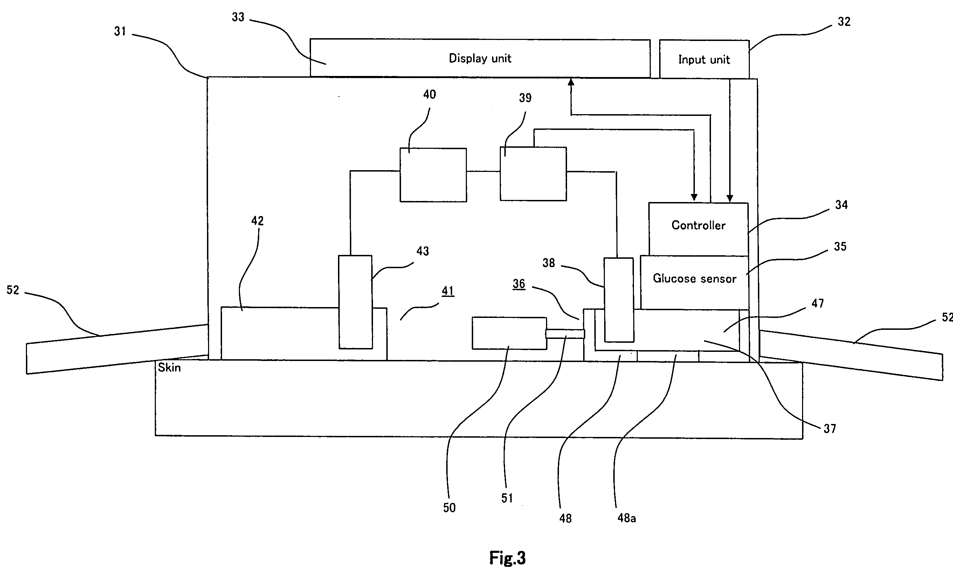

Fig. 3 is a block diagram briefly showing the structure of the blood sugar measuring device 31. The blood sugar measuring device 31 is provided with an input unit 32 for inputting an initial blood sugar value described later, display unit 33 for displaying input values and measurement results, control unit 34 for controlling the operation of the blood sugar measuring device 31, calculating glucose concentration, calculating blood sugar values and the like, glucose sensor 35 for detecting glucose, extraction unit 36 which is positioned on the extraction region 22 for collecting glucose ammeter 39. constant-voltage power source 40, positive electrode unit 41, syringe 50, and band 52. The extraction unit 36 is provided with a fluid chamber 37 and negative electrode 38. The fluid chamber 37 includes physiological saline 47 for collecting glucose extracted from a living body through the skin, and a chamber 48 for accommodating the physiological saline 47. The negative electrode 38 is immersed in the physiological saline 47. The chamber 48 has an aperture 48a which allows the physiological saline 47 to contact the extraction region 22 of the skin. The physiological saline 47 is injected from a syringe 56 into the chamber 48 through a flow path 51 after the blood sugar measuring device 31 has been placed on the wrist of the subject.

The negative electrode 38 is connected to the negative pole side of the constant-voltage power source 40 through the ammeter 39, and the positive electrode unit 41 is connected to the positive pole side of the constant-voltage power source 40. The positive electrode unit 41 is provided with gel 42, and a positive electrode 43 immersed in the gel 42. The positive electrode 43 is connected to the positive pole side of the constant-voltage power source 40.

The glucose sensor 35 is a sensor that generates signals based on substance generated by a reaction between glucose and oxygen. For example, the sensor disclosed in US Patent Application Publication No. 2003-225322 may be used as the glucose sensor.

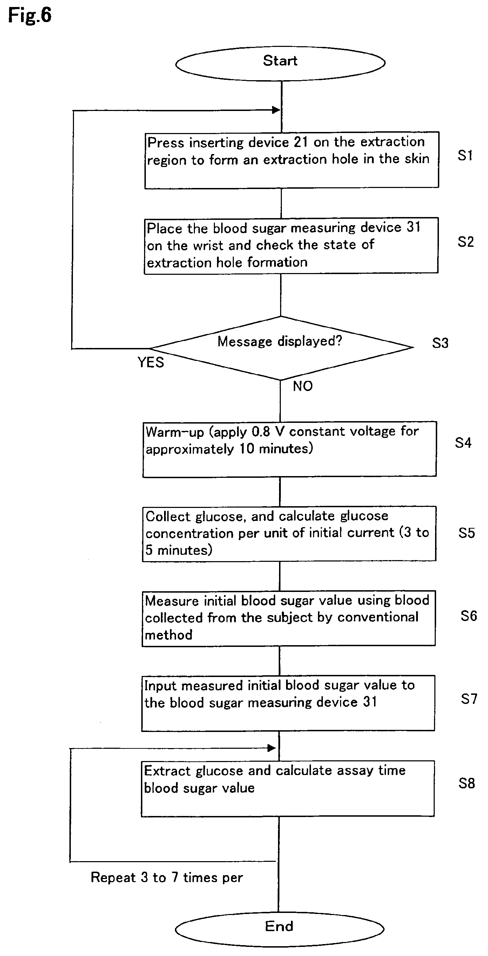

The extracting kit of the present embodiment is described according to its usage sequence. Fig. 6 is a flow chart showing the operating sequence performed each day by the subject using the extracting kit. First, in step S1, the subject lightly presses the inserting device 21 on the skin in the vicinity of the writ and forms the extraction hole, as shown in Fig. 1(a). The region on the surface of the skin where the extraction hole is formed is the extraction region 22. At this stage, the marker 25 may be adhered as a marker to the extraction region 22 in which the extraction hole is formed for later repeat measurement, as shown in Fig. 1(c). The marker 25 is a sheet having an opening of a predetermined size; the marker 25 is adhered to the skin such that the position of the opening matches the position of the extraction region 22.

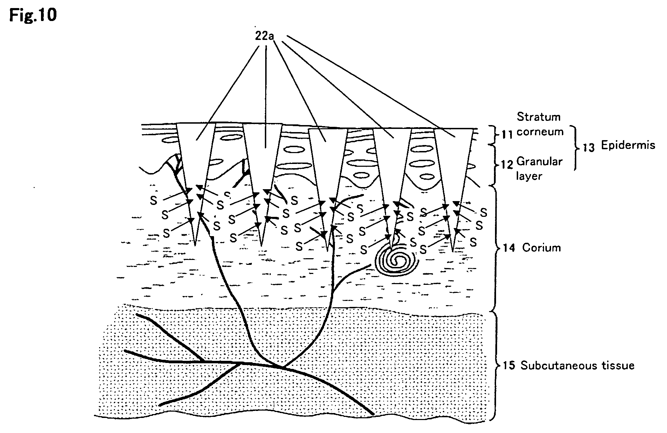

As shown in Fig. 10, the plural extraction holes 22a formed in the extraction region 22 in Step S1 penetrate through the stratum corneum 11 and the granular layer and others 12, reaching close to the middle of the corium 14 but not the subcutaneous tissue 15. Each of the extraction holes 22a has the largest diameter at the skin surface and a smaller diameter as the hole approaches to the subcutaneous tissue 15. The diameter of the extraction hole 22a at the skin surface is approximately 160 µm and the depth of the extraction hole 22a is approximately 300 µm. After extraction holes 22a are formed in Step S1, the body fluid in the corium 14 is extracted into the extraction hole 22a, as indicated by arrows S. The body fluid contains glucose.

The pressing of the inserting device 21 on the skin in step S1 is performed for the purpose of forming the extraction hole and does not require that the needles puncture the skin during the period of glucose extraction. Accordingly, the time during which the inserting device 21 is inserted into the skin is relatively short compared to needles of the conventional art.

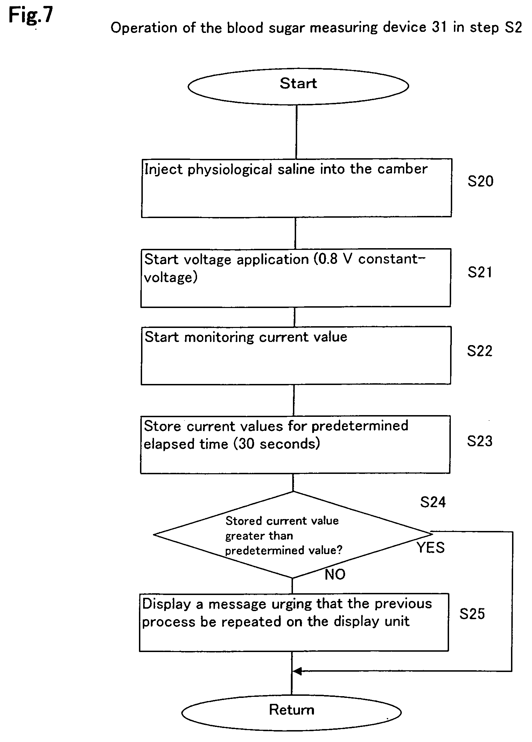

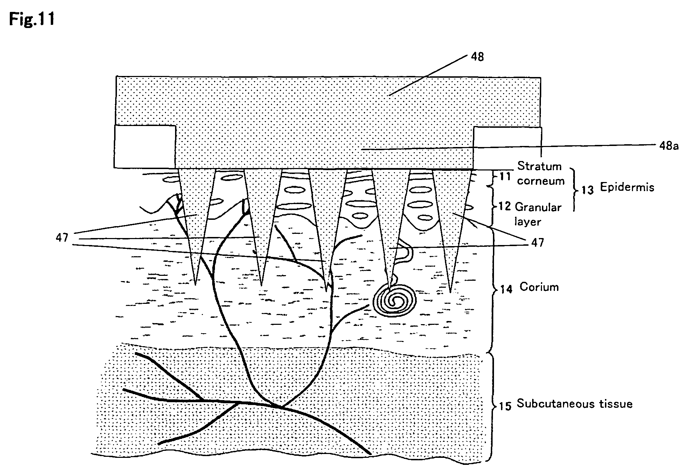

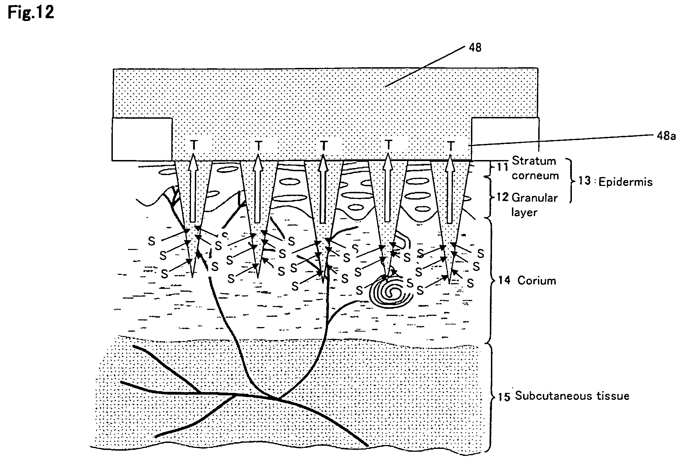

After the inserting device 21 is removed from the skin, the blood sugar measuring device 31 is placed on the wrist of the subject and fastened in placed using the band 52 in step S2, as shown in Fig. 1(b). In this way the blood sugar measuring device 31 is placed with the fluid chamber 37 disposed on the extraction region 22, as shown in Fig. 3. Then, physiological saline 47 is injected into the chamber 48 via a flow path 51 from a syringe 50. Then, an electric current is supplied from the constant-voltage power source 40 and flows through the positive electrode, living body, and negative electrode, that is an electric field is applied to the living body, and the state of the extraction hole is checked. Fig. 7 shows details of the operation of the blood sugar measuring device 31 in step S2.
In Step S20, the physiological saline 47 is injected from the syringe 50 via the flow path 51 into the chamber 48 by the operation of syringe 50 by the subject. The physiological saline 47 injected into the chamber 48 becomes in contact with the skin extraction region 22a via an aperture 48a. The physiological saline 47 that has been brought into contact with the extraction region 22a flows into the extraction hole 22a, as shown in Fig. 11. As shown in Fig. 12, upon influx of the physiological saline 47 into the extraction hole 22a, the body fluid extracted into the physiological saline 47 present in extraction hole 22a formed in Step S1 migrates toward physiological saline 47 present in the chamber 48 (in the T direction in Fig. 12). As the concentration of the body fluid becomes lower in the physiological saline 47 inside the extraction holes 22a, the body fluid is then further extracted from the corium 14 to the physiological saline 47 in the extraction hole 22a, as indicated by arrows S.
As shown in step S21, a 0.8 V constant-voltage current is supplied from the constant-voltage power source 40. In step S22, the ammeter 39 (Fig. 3) begins monitoring the current value. In step S23, the current value for a 30 second period are stored in the control unit 34. In step S24, the control unit 34 determines whether or not the current value stored in the control unit 34 exceeds a predetermined value; if the predetermined value is exceeded, the process moves to step S3, whereas when the predetermined value is not exceeded, a message urging that the process (formation of the extraction hole by the inserting device 21) be performed again is displayed on the display unit 33 (step S25), and the process moves to step S3. The steps S21 through step S25 are performed by the control unit 34.

In step S3, the subject conformed whether or not a message urging that the previous process be repeated is displayed on the display unit 33. If such a message is not displayed on the display unit 33, the blood sugar measuring device 31 automatically executes the process of step S4. When the message is displayed, the subject removes the blood sugar measuring device 31 from the wrist, and again performs the process of step S1.

Then, in step S4, a 0.8 V constant-voltage is applied from the constant-voltage power source 40 to the living body in an approximately 10 minute warm-up period. This warm-up allows the extraction fluid (physiological saline) within the fluid chamber 37 to make sufficient contact with the skin, avoids the influence of the needles 23 damaging the skin cells and stabilizes the amount of extracted glucose. Step S4 is not necessarily required.

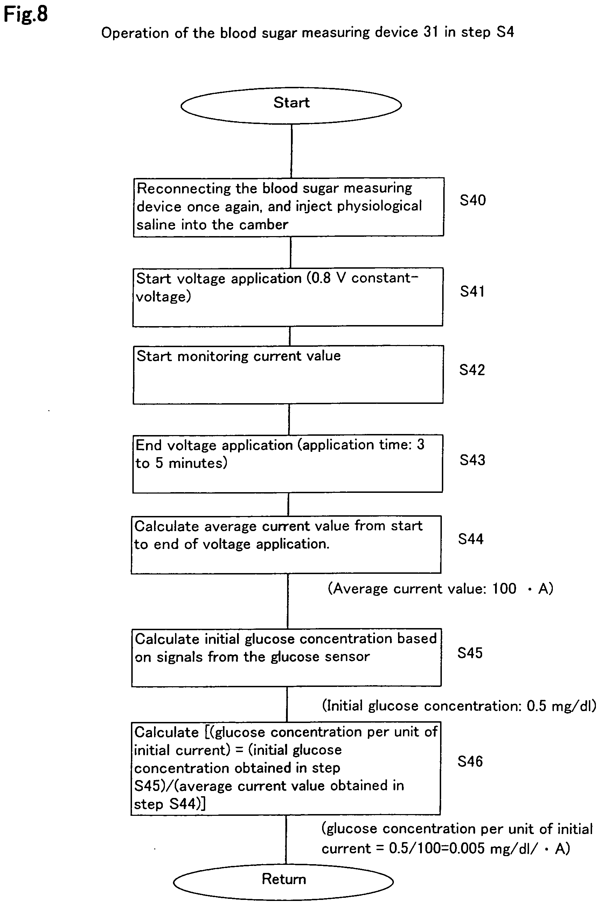

Next, in step S5, collection of glucose in the chamber 48 is performed, and initial glucose concentration, average current value, and glucose concentration per unit of initial current value are calculated. Fig. 8 shows details of the operation of the blood sugar measuring device 31 in step S5.
In step S40, the subject removes the blood sugar measuring device 31 from the wrist, exchanges the extraction unit 36 with new one, reconnects the blood sugar measuring device 31 once again to the same position of the wrist, and injects physiological saline 47 from the syringe 50 via the flow path 51 into the chamber 48. As a result, in a similar manner to Step S20, the body fluid extracted into the extraction holes 22a migrates (diffuses) toward the physiological saline 47 present in the chamber 48 (in the T direction in Fig. 12), as shown in Fig. 12. As the concentration of the body fluid in the physiological saline 47 inside the extraction holes 22a becomes lower, the body fluid is then further extracted from the corium 14 to the physiological saline 47 in the extraction hole 22a, as indicated by arrows S.

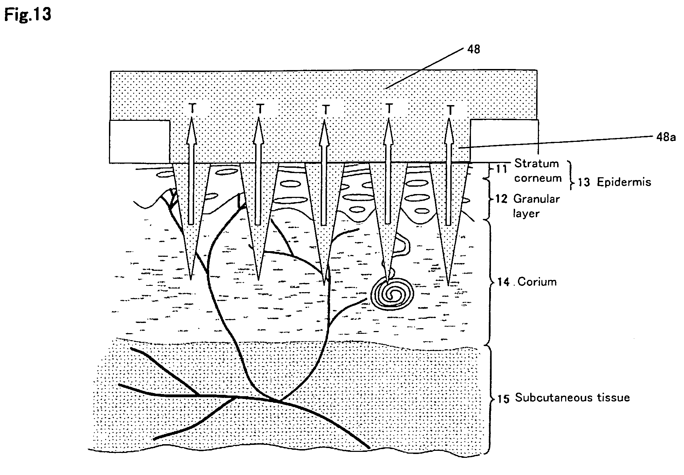

In step S41, a 0.8 V constant-voltage current is supplied from the constant-voltage power source 40, that is, an electric field is applied to the living body, and in step S42 the ammeter 39 (Fig. 3) begins monitoring the current value.
The body fluid extracted into the physiological saline 47 in the extraction holes 22a, which is carrying charges, is driven to migrate toward the chamber 48 (in the T direction in Fig. 13) by application of an electric field in S41, as shown in Fig. 13. Although carrying no charge, glucose contained in the body fluid also migrates together with the other components carrying charges.

The 0.8 V voltage is set such that a current is concentrated and flows only in the extraction hole, and current effectively does not flow in the stratum corneum outside the extraction hole. It is desirable that the voltage supplied from the constant-voltage power source 40 to the living body is less than approximately 10 V from the perspective of setting the current to effectively not flow in the stratum corneum of the skin. Furthermore, it is desirable that the voltage is greater than 0.05 V from the perspective of collecting an amount of glucose required for analysis in the chamber 48 and improving analysis accuracy.

After 3 to 5 minutes have elapsed, the current supply is stopped in step S43, and in step S44 the average current value from the start to the end of the current application, that is, the average current value during the initial glucose concentration calculation, is calculated. In one embodiment, the average current value is 100 µA.

Then, in step S45, the control unit 34 calculates the initial glucose concentration based on signals from the glucose sensor 35 (Fig. 3). In one embodiment, the initial glucose concentration is 0.5 mg/dl.

In step S46, the glucose concentration per unit of initial current is calculated. The glucose concentration per unit of initial current is obtained by dividing the initial glucose concentration obtained in step S45 by the average current value obtained in step S44. Accordingly, in one of the aforesaid embodiments, the glucose concentration per unit of initial current is 0.5/100=0.005 mg/dl. After step S46 ends, the process moves to step S6. The steps S41 through step S46 are performed by the control unit 34.

In step S6, the subject determines the initial blood sugar value as the blood sugar value before measuring the blood sugar value using the blood sugar measuring device 31 by collecting blood from a fingertip using a conventional blood sugar measuring device (for example, the Freestyle produced by Nipro K.K.). In one embodiment, the initial blood sugar value is 80 mg/dl. The initial blood sugar value determined in this way is input from the input unit 32 (Fig. 3) in step S7. Hereinafter, measurement of blood sugar value is accomplished using the blood sugar measuring device 31 of the present embodiment.

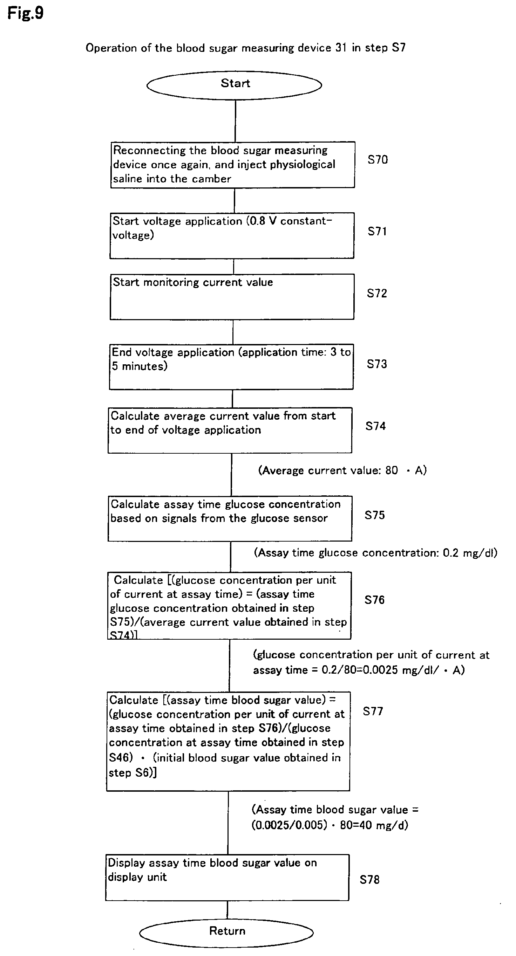

Step S8 shows the operation when the blood sugar value is measured using the blood sugar measuring device 31 of the present embodiment after a predetermined time has elapsed in order to see the change in the blood sugar value of one day, for example. Fig. 9 shows details of the operation of the blood sugar measuring device 31 in step S8.
In step S70, the subject removes the blood sugar measuring device 31 from the wrist, exchanges the extraction unit 36 with new one, reconnects the blood sugar measuring device 31 once again to the same position of the wrist, and injects physiological saline 47 from the syringe 50 via the flow path 51 into the chamber 48. Thus, in a similar manner to Step S20, the body fluid extracted into the extraction holes 22a migrates (diffuses) toward the physiological saline 47 present in the chamber 48 (in the T direction in Fig. 12), as shown in Fig. 12. As the concentration of the body fluid in the physiological saline 47 in the extraction holes 22a becomes lower, the body fluid is then further extracted from the corium 14 to the physiological saline 47 in the extraction hole 22a, as shown by arrows S.

In step S71, a 0.8 V constant-voltage current is supplied from the constant-voltage power source 40, that is, an electric field is applied to the living body, and in step S72 the ammeter 39 (Fig. 3) begins monitoring the current value.
The body fluid extracted into the physiological saline 47 in the extraction holes 22a, which is carrying charges, is driven to migrate toward the chamber 47 (in the T direction in Fig. 13) by application of an electric field in S71, as shown in Fig. 13.
After 3 to 5 minutes have elapsed, the current supply is stopped in step S73, and in step S74 the average current value from the start to the end of the current application, that is, the average current value during the assay time glucose concentration calculation, is calculated. In one embodiment, the average current value is 80 µA.

Then, in step S75, the control unit 34 calculates the assay time glucose concentration based on signals from the glucose sensor 35 (Fig. 3). In one embodiment, the assay time glucose concentration is 0.2 mg/dl.

In step S76, the glucose concentration per unit of current at assay time is calculated. The glucose concentration per unit of current at assay time is obtained by dividing the assay time glucose concentration obtained in step S75 by the average current value obtained in step S74. Accordingly, in one of the previously mentioned embodiments, the obtained glucose concentration per unit of current at assay time is 0.2/80=0.0025 mg/dl/µA.

Then, in step S77, the assay time blood sugar value is calculated. The assay time blood sugar value is calculated by dividing the glucose concentration per unit of current at assay time obtained in step S76 by the glucose concentration per unit of initial current, and multiplying by the initial blood sugar value input in step S6. Accordingly, on one embodiment, the assay time blood sugar value is 40 mg/dl [(0.0025/0.005)x80].

Finally, in step S78, the assay time blood sugar value determined in step S77 is displayed on the display unit 33 (Fig. 3), and one cycle of the measurement of the blood sugar value ends.
The measurement of the blood sugar value in step S8 using the blood sugar measuring device 31 of the present embodiment can be performed several times over the course of one day.

Although the average current value is used in steps S44 and S74 of the present embodiment, the present invention is not limited to this arrangement inasmuch as various other current values may be used, such as the current value of a predetermined time course, maximum current value within a predetermined time and the like.

The extracting kit according to the present embodiment allows extraction of the analyte practically without causing pain in the subject, by forming extraction holes by the inserting device 21 that reaches the corium 14 but not the subcutaneous tissue 15, supplying a liquid to the extraction holes, and extracting an analyte from the body. In addition, the extracting device according to the present embodiment allows efficient collection of the analyte extracted in the extraction holes in the extraction unit 36, practically without causing pain in the subject by supplying a weak current to the liquid in the extraction holes from the constant-voltage power source 40.

Furthermore, since the inserting device 21 does not reach the subcutaneous tissue where the presence of nerves and the like would enhance the painful sensation, the subject feels no pain, and the physical and psychological burdens on the subject are reduced. Since the time during which the inserting device 21 is inserted in the skin is short compared to conventional needles, there is less concern of the needle 23 breaking the inner part of the skin, such that damage to the skin is also reduced. In addition, the risk of infection and the like is reduced since the time during which the inserting device 21 is inserted in the skin is short compared to conventional needles.

Since the constant-voltage power source 40 applies to a living body a voltage of a magnitude such that a current is concentrated and flows only in the extraction hole and the current does not actually flow in the region of the stratum corneum outside the extraction hole, the number and condition of the extraction holes formed by the inserting device 21 is unchanged by the application of the voltage, and the extraction of analyte can be performed under identical conditions multiple times over time from the once-formed extraction hole.

Furthermore, since the glucose concentration per unit of initial current and the glucose concentration per unit of current at assay time are determined to compensate the blood sugar value in the present embodiment, the measurement is unaffected by a change over time in the number and condition of the extraction hole and a blood sugar measurement of high accuracy can be obtained even when the number and condition of the extraction hole changes over time.

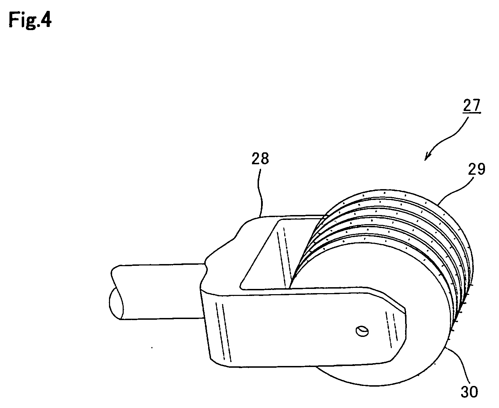

Fig. 4 is a perspective view showing another embodiment of the inserting device 21. The needle roller 27 shown in Fig. 4 may be used as the inserting device 21. The needle roller 27 has an arm 28, and a roller 29 supported by the arm 28 so as to be rotatable; an array of a plurality of needles 30 are maintained at equal distances on the circumference aligned parallel to the rotational axis of the roller 29. When the needle roller 27 is used, a plurality of extraction holes are formed in a relatively wide area of the skin. An example of a useful needle roller 27 is the Derma Roller (Top-Rol K.K.).

In consideration of the change in the condition of the extraction hole over time in the above embodiments, the embodiment has been described in terms of determining the assay time blood sugar value using an initial blood sugar value, glucose concentration per unit of initial current obtained by dividing the initial glucose concentration by an average current value, and the glucose concentration per unit of current at assay time obtained by dividing the assay time glucose concentration by an average current value, however, the assay time blood sugar value also may be determined using the initial blood sugar value, initial glucose concentration, and assay time glucose concentration without using the current value when there is little change in the condition of the extraction hole over time. In this case, the relationship [assay time blood sugar value = (assay time glucose concentration/initial glucose concentration) x initial blood sugar value] is used. Also in this instance measurement errors based on the condition of the skin characteristics of the subject are eliminated.

When the condition of the intrinsic skin characteristics of the subject are ignored in the above embodiments, the value (blood sugar value/glucose concentration) is a value characteristics of the extracting kit used under identical conditions. Accordingly, when the value (blood sugar value/glucose concentration) is input to the control unit 34 beforehand as a parameter, the assay time blood sugar value can be determined by multiplying the parameter and the calculated assay time glucose concentration.

Although the measurement of the initial blood sugar value is performed after the measurement of the initial glucose concentration in the above embodiments, the blood sugar measurement also may be performed simultaneously or before the initial glucose concentration measurement insofar as such measurement are not separated by a lengthy time. Furthermore, although the glucose extraction is performed for 3 to 5 minutes, the time may be suitably modified in accordance with measurement accuracy and the situation.

The above embodiments have been described in terms of a structure for determining the state of extraction hole formation by monitoring a current value using a constant-voltage power source as a power supply; however, a structure is also possible in which the state of extraction hole formation is determined by monitoring a voltage value using a constant-current power source.

In the case of the above structures, the control unit 34 supplies a constant current in step S21, that is, starts the application of an electric field to the living body, begins monitoring the voltage value in step S22, stores the time-course voltage values for a predetermined time (30 seconds) in step S23, and determines whether or not the stored voltage value exceeds a predetermined value in step S24. A process is executed in step S25 when the stored voltage value exceeds a predetermined value, and a process is executed in step S3 when the voltage value does not exceed the predetermined value.

Although the above embodiments have been described in terms of extracting analyte using a constant-voltage power source as a power supply, and determining the glucose concentration per unit of initial current, and glucose value per unit of current at assay time, alternatively, analyte may be extracted using a constant-current power source as a power supply and voltmeters may be provided for monitoring the voltage at both ends of the power source, so as to determine the glucose concentration per unit conductivity (=glucose concentration/average conductivity). That is, conductivity = [current value (constant)/voltage value].

In such a structure, the average conductivity is calculated from the start to the end of the current application in step S44, and in step S46 the glucose concentration per unit of initial conductivity is calculated by dividing the initial glucose concentration obtained in step S45 by the average conductivity obtained in step S44.

Furthermore, the average conductivity is calculated from the start to the end of the current application in step S74, and the glucose conductivity per unit of conductivity at assay time is calculated in step S76 by dividing the assay time glucose concentration obtained in step S75 by the average conductivity obtained in step S74. Then, in step S77, the assay time blood sugar value is calculated by dividing the glucose concentration per unit of conductivity at assay time obtained in step S76 by the glucose concentration per unit of initial conductivity, and multiplying by the initial blood sugar value input in step S7.

In the embodiments above, described is an extracting device that collects the analyte extracted in the extraction holes in the extraction unit 36 by supplying a weak current to the liquid in the extraction holes from a constant-voltage power source 40, but the present invention is not limited to this arrangement inasmuch as the present invention also may be applied to an extraction device for collecting glucose without applying an electric field to a living body. In this case the extracting device also may apply an electric field to a living body only to check the state of the extraction hole formation.

In addition, although an extracting device that collects the analyte extracted in the extraction hole into the extraction unit 36 by supplying a weak current to the liquid in the extraction hole by a constant-voltage power source 40 is described in the embodiments above, the present invention also may be applied to extracting devices for collecting analyte extracted in the extracting hole in the extracting unit 36 by irradiating the extracting hole with ultrasound, and extracting devices for collecting analyte extracted in the extracting hole in the extracting unit 36 by applying a negative pressure to the extracting hole.

Although the above embodiments have been described in terms of a blood sugar measuring device for extracting glucose and calculating a blood sugar value, the present invention is not limited to this arrangement inasmuch as the present invention is also applicable to extracting devices for extracting analytes other than glucose such as biochemical substances and drugs administered to the subject.

Further, although physiological saline was used as the liquid to be supplied into the extraction holes in the embodiments above, the present invention is not limited to physiological saline, and may employ a conductive liquid other than physiological saline or a nonconductive liquid such as pure water.
Further, although the extraction unit 36 had a negative electrode 38 and the anodal unit 41 had a positive electrode 43 in the embodiments above, the present invention is not limited thereto, the extraction unit 36 may have both the negative electrode 38 and the positive electrode 43. In such a case, both the negative electrode 38 and the positive electrode 43 become in contact with the physiological saline 47.

## Claims

1. An extracting kit for extracting analyte through the skin of a living body, the extracting kit comprising:
an inserting device comprising needles for forming a plurality of extraction holes in the skin through the stratum corneum but not reaching the subcutaneous tissue; and
an extracting device for extracting the analyte into a liquid supplied into the extraction holes by supplying the liquid into the plurality of extraction holes.

2. The extraction kit of Claim 1, wherein the extracting device further comprises:
a liquid holder for retaining the liquid to supply into the extraction holes;
a sensor placed in contact with the liquid retained in the liquid holder; and
a power source for applying a voltage for migrating the analyte extracted into the liquid supplied to the extraction holes toward the sensor.

3. The extracting kit of Claim 2, wherein the power source applies to the living body a voltage of a magnitude such that a current does not actually flow to the stratum corneum of the skin.

4. The extracting kit of Claim 3, wherein the power source is a constant-voltage power source for applying a constant voltage greater than approximately 0.5 V but less than approximately 10 V.

5. The extracting kit of Claim 1, wherein the length of the part of the needle inserted into the skin is greater than approximately 20 µm but less than approximately 1,000 µm.

6. The extracting kit of Claim 1, wherein the inserting device comprises the plurality of needles and a holder capable of holding the plurality of needles.

7. The extracting kit of Claim 1, wherein the extracting device comprises, a power source, negative and positive electrodes connected to the power source, and a collection medium provided in contact with at least one or another of the negative electrode and positive electrode so as to collect the extracted analyte.

8. The extraction kit of Claim 1, further comprising: a marker for specifying the position on the skin at which the extraction hole is formed.

9. The extraction kit of Claim 1, wherein the extracting device further comprises a checking means for checking the state of extraction hole formation.

10. The extracting kit of Claim 1, wherein the extracting device further comprises a power source for applying an electric field to the living body for checking the state of extraction hole formation.

11. The extracting kit of Claim 1, wherein the extracting device further comprises an analyzing means for analyzing the extracted analyte.

12. The extracting kit of Claim 1, wherein the extraction hole in the skin passes through the stratum corneum and reaches corium but does not reach the subcutaneous tissue.

13. An extraction device for extracting analyte through the skin of a living body, the extraction device comprising:
a power source for applying a voltage for transferring analyte extracted into extraction holes formed on the skin out of the extraction holes; and
a checking means for checking the state of extraction hole formation.

14. The extraction device of Claim 13, wherein the checking means checks the state of extraction hole formation based on a current value when the power source applies an electric field to the living body.

15. The extracting device of Claim 14, wherein the checking means monitors the current value when the power source applies an electric field to the living body, and determines the extraction hole has not been adequately formed when the current value is less than a predetermined value.

16. The extracting device of Claim 13, wherein the checking means checks the state of extraction hole formation based on the voltage value when the power source applies an electric field to the living body.

17. The extracting device of Claim 16, wherein the checking means monitors the voltage value when the power source applies an electric field to the living body, and determines the extraction hole has not been adequately formed when the voltage value is less than a predetermined value.

18. The extracting device of Claim 13, further comprising:
an outputting means for outputting a message urging extraction hole formation when the checking means determines that the extraction hole has not been adequately formed.

19. The extracting device of Claim 13, wherein the extraction hole passes through the stratum corneum but does not reach the subcutaneous tissue.

20. A blood sugar measuring device for extracting glucose through skin of a living body and analyzing the glucose, the measuring device comprising:
an input unit for inputting an initial blood sugar value;
a power source for transferring glucose extracted into extraction holes formed on the skin out of the extraction holes;
a detecting unit for detecting signals based on the extracted glucose; and
a control unit for calculating a blood sugar value from the signal detected by the detecting unit;
wherein the control unit calculates glucose concentration from the signal detected by the detecting unit, and calculates an assay time blood sugar value based on the glucose concentration and the initial blood sugar value input by the input unit.

21. The blood sugar measuring device of Claim 20,
wherein the control unit calculates the assay time blood sugar value based on the initial blood sugar value, initial glucose concentration when the initial blood sugar value was input, and the assay time glucose concentration when calculating the assay time blood sugar value.

22. The blood sugar measuring device of Claim 20,
wherein the control unit determines the glucose concentration per unit of initial current based on current value and initial glucose concentration when the initial blood sugar value is input, determines the glucose concentration per unit of current at assay time based on current value and assay time glucose concentration when the assay time blood sugar value is calculated, and calculates the assay time blood sugar value based on the glucose concentration per unit of initial current and the glucose concentration per unit of current at assay time and the initial blood sugar value.

23. The blood sugar measuring device of Claim 20,
wherein the control unit determines the glucose concentration per unit of initial conductivity based on voltage value and initial glucose concentration when the initial blood sugar value is input, determines the glucose concentration per unit of conductivity at assay time based on voltage value and assay time glucose concentration when the assay time blood sugar value is calculated, and calculates the assay time blood sugar value based on the glucose concentration per unit of initial conductivity and the glucose concentration per unit of conductivity at assay time and the initial blood sugar value.

24. The blood sugar measuring device of Claim 20,
wherein the extraction hole passes through the stratum corneum but does not reach the subcutaneous tissue.

25. An extracting method for extracting analyte through the skin of a living body, the method comprising:
an extraction hole formation step of forming a plurality of extraction holes in the skin through the stratum corneum but not reaching the subcutaneous tissue; and
an extraction step of extracting the analyte into a liquid supplied to the extraction holes by supplying the liquid into the plurality of extraction holes.

26. The extracting method of Claim 25, further comprising a transferring step of transferring the analyte extracted into the liquid supplied to the extraction holes out of the extraction holes by applying a voltage to the liquid supplied to the extraction holes.

27. The extracting method of Claim 25, wherein, in the transferring step, the voltage applied to the living body is of a magnitude such that a current does not actually flow to the stratum corneum of the skin.

28. The extracting method of Claim 27, wherein the voltage is a constant voltage greater than approximately 0.5 V but less than approximately 10 V.

29. The extracting method of Claim 25, wherein the extraction hole is formed using a needle, the part of which that is inserted into the skin has a length that is greater than approximately 20 µm but less than approximately 1,000 µm.

30. The extracting method of Claim 26, further comprising:
a step of placing an extraction device for extraction of the analyte on the skin, and
a collection step of collecting the analyte transferred out of the extraction holes in the transferring step.

31. The extracting method of Claim 25, further comprising:
a step of placing a marker on the skin to specify the position on the skin at which the extraction hole is formed.

32. The extracting method of Claim 25, further comprising:
a checking step for checking the state of extraction hole formation after the extraction hole formation step has been executed.

33. The extracting method of Claim 32, wherein the extraction hole formation step is executed again when it has been determined that extraction hole formation is inadequate in the checking step.

34. The extraction method of Claim 32, further comprising:
an electric field application step for applying an electric field to the living body to check the state of extraction hole formation.

35. The extracting method of Claim 25, further comprising:
a warm-up step for stabilizing the amount of extracted analyte.

36. The extracting method of Claim 25, further comprising:
an analysis step for analyzing the analyte extracted in the extraction step.

37. The extracting method of Claim 36, wherein the analysis step includes a step of detecting the extracted glucose and calculating blood sugar value.

38. The extracting method of Claim 25, wherein the extraction hole formed in the extraction hole formation step passes through the stratum corneum and reaches corium but does not reach the subcutaneous tissue.

39. An extracting method for extracting analyte through the skin of a living body, the method comprising:
an extraction hole formation step for forming a plurality of extraction holes in the skin for the extraction of analyte;
a checking step for checking the state of extraction hole formation; and
a transferring step of transferring the analyte extracted into the extraction holes out of the extraction holes if the extraction holes are judged to be formed sufficiently in the checking step.

40. The extracting method of Claim 39, wherein the checking step comprises an electric field application step for applying an electric field to the living body, and a current checking step for checking the state of extraction hole formation based on current value in the electric field application step.

41. The extracting method of Claim 40, wherein, in the current checking step, the extraction hole formation is determined to be inadequate when the current value is less than a predetermined value in the electric field application step.

42. The extracting method of Claim 39, wherein the checking step comprises an electric field application step for applying an electric field to the living body, and a voltage checking step for checking the state of extraction hole formation based on voltage value in the electric field application step.

43. The extracting method of Claim 42, wherein, in the voltage checking step, the extraction hole formation is determined to be inadequate when the voltage value exceeds a predetermined value in the electric field application step.

44. The extracting method of Claim 39, further comprising:
an output step for outputting a message urging extraction hole formation when it is determined the extraction hole formation is inadequate in the checking step.

45. The extracting method of Claim 39, wherein the extraction hole formed in the extraction hole formation step passes through the stratum corneum but does not reach the subcutaneous tissue

46. A blood sugar measuring method for extracting glucose through the skin of a living body and analyzing the glucose, the method comprising:
an extraction hole formation step for forming an extraction hole in the skin for extracting analyte;
an initial blood sugar value input step for inputting an initial blood sugar value;
an electric field application step of applying a voltage for migrating glucose extracted into the extraction hole out of the extraction hole;
a detection step for detecting a signal based on the glucose extracted in the extracting device; and
a calculation step for calculating a blood sugar value from a signal detected in the detection step; wherein, in the calculation step, a glucose concentration is calculated from the signal detected in the detection step, and an assay time blood sugar value is calculated based on the glucose concentration and an initial blood sugar value input in the initial blood sugar value input step.

47. The blood sugar measuring method of Claim 46,
wherein in the calculation step, the assay time blood sugar value is calculated based on the initial blood sugar value, the initial glucose concentration when the initial blood sugar value was input, and the assay time glucose concentration when the assay time blood sugar value was calculated.

48. The blood sugar measuring method of Claim 46,
wherein, in the calculation step, the glucose concentration per unit of initial current is determined based on current value and initial glucose concentration when the initial blood sugar value is input, the glucose concentration per unit of current at assay time is determined based on current value and assay time glucose concentration when the assay time blood sugar value is calculated, and the assay time blood sugar value is calculated based on the glucose concentration per unit of current at assay time, the glucose concentration per unit of initial current and the initial blood sugar value.

49. The blood sugar measuring method of Claim 46,
wherein, in the calculation step, the glucose concentration per unit of initial conductivity is determined based on voltage value and initial glucose concentration when the initial blood sugar value is input, the glucose concentration per unit of conductivity at assay time is determined based on voltage value and assay time glucose concentration when the assay time blood sugar value is calculated, and the assay time blood sugar value is calculated based on the glucose concentration per unit of initial conductivity, the glucose concentration per unit of conductivity at assay time and the initial blood sugar value.

50. The blood sugar measuring device of Claim 46,
wherein the extraction hole formed in the extraction hole formation step passes through the stratum corneum and reaches corium but does not reach the subcutaneous tissue.

51. An extracting kit for extracting analyte through the skin of a living body, the extracting kit comprising:
an inserting device comprising a needle for forming an extraction hole in the skin through the stratum corneum but not reaching the subcutaneous tissue; and
an extracting device for extracting analyte outside of the extraction hole and placed on the skin where the extraction hole is formed.

52. An extracting method for extracting analyte through the skin of a living body, the method comprising:
an extraction hole formation step of forming an extraction hole in the skin through the stratum corneum but not reaching the subcutaneous tissue; and
an extraction step of extracting analyte outside of the extraction hole.
